(19) Europäisches Patentamt / European Patent Office / Office européen des brevets

(11) EP 2 705 771 A1

(12) **EUROPEAN PATENT APPLICATION**

(43) Date of publication:
**12.03.2014 Bulletin 2014/11**

(21) Application number: **12183441.0**

(22) Date of filing: **07.09.2012**

(51) Int Cl.:
***A45D 19/00*** *(2006.01)* ***A61K 8/19*** *(2006.01)*
***A61K 8/34*** *(2006.01)* ***A61K 8/37*** *(2006.01)*
***A61Q 5/10*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Unilever PLC**
**London, Greater London EC4Y 0DY (GB)**

(72) Inventor: **The designation of the inventor has not yet been filed**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(54) **Hair care composition**

(57) A kit for colouring hair comprising at least two compositions and a package containing the compositions in which:
a) the package comprises:
(i) a separate reservoir for each composition
(ii) a dispensing outlet for dispensing a first composition from a first reservoir and a second dispensing outlet for dispensing a second composition from a second reservoir; and in which
(iii) the dispensing outlet of the first reservoir is positioned at an angle from 90 to 270 degrees on a horizontal plane from the dispensing outlet of the second reservoir;

b) the compositions comprise:
(i) a first composition comprising a polyphenol based formulation;
(ii) a second composition comprising a metal ion based formulation in which the first composition is contained within the first reservoir and the second composition is contained within the second reservoir.

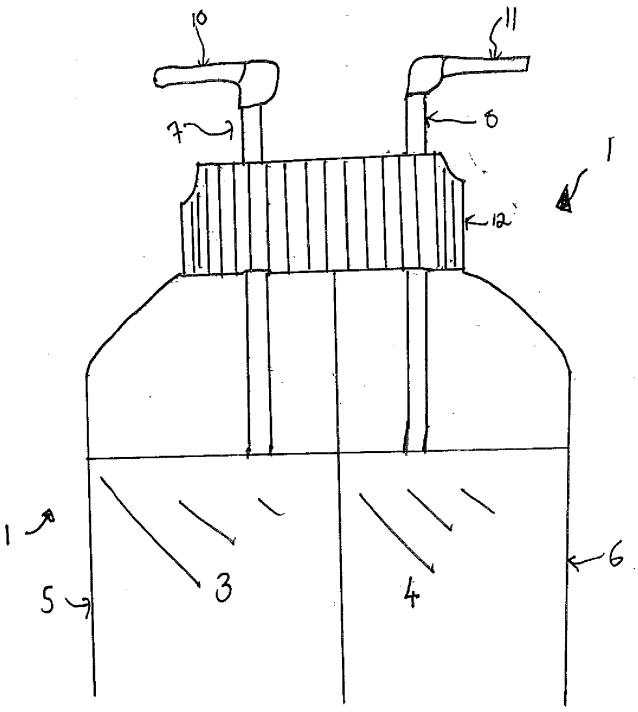


EP 2 705 771 A1

## Description

The present invention relates to hair colourants and packaging.

Background

The present invention relates to a hair colouring system. Convention permanent colouring systems having more than one formulation type are usually mixed together prior to application to the hair. However such system can stain the skin of the hands during application and are usually applied to the hair using gloves.

The present application relates to a permanent hair colouring system, that mitigates the colouring of the hands during application. The colouring system of the present invention involves application of compositions that are added sequentially to the hair. To avoid staining of the hands the products should not be mixed together.

As the consumer has an expectation to mix hair formulations during the hair colouring process a package that discourages intermixing of the compositions leads to a decrease in staining of the hands during the colouring process.

Description

Accordingly the present invention relates to a kit for colouring hair a kit for colouring hair comprising at least two compositions and a package containing the compositions in which:

a) the package comprises:

i) a separate reservoir for each composition
ii) a dispensing outlet for dispensing a first composition from a first reservoir and a second dispensing outlet for dispensing a second composition from a second reservoir; in which
iii) the dispensing outlet of the first reservoir is positioned at an angle from 90 to 270 degrees on a horizontal plane from the dispensing outlet on the second reservoir;

b) the compositions comprise:

(i) a first composition comprising a polyphenol based formulation;
(ii) a second composition comprising a metal ion based formulation; in which the first composition is contained within the first reservoir

and the second composition is contained within the second reservoir.

The invention also relates to a process for colouring hair, the process using the kit of any of the preceding claims, the process comprising the steps of:

(i) dispensing a first composition from the first reservoir using the first dispensing outlet;
ii) applying the composition to the hair;
iii) rinsing the hair;
iv) dispensing a second composition from the second reservoir using the second dispensing outlet;
v) applying the composition to the hair;
vi) rinsing the hair.

The hair may be wet or dry at the start of the process.

Package

The kit according to the invention contains a package having at least two reservoirs for two compositions. Each composition is contained in a separate reservoir. The packaging may comprise two separate containers bound or packaged together. Alternatively the package may comprise at least two separate chambers/reservoirs in one container. Preferably the package comprises a bottle.

The package comprises two reservoirs having separate dispensing outlets. Preferably the first dispensing outlet is static relative to the second dispensing outlet. As the dispensing outlets do not move relative to each other there is no possibility of cross contamination of compositions leading to discolouration on the package and on the hands when in use.

Preferably each reservoir has a volume from 20 to 500 ml. most preferably 50 to 250ml. Preferably the ratio of the volume of the first reservoir to the second reservoir is from 1:1 to 1:3.

The dispensing outlet of the first reservoir is positioned at an angle from 90 to 270 degrees on a horizontal plane from the dispensing outlet on the second reservoir preferably at an angle from 160 to 200 degrees.

It is preferred if each dispensing outlet comprises a hand-operated pump actuator by which the formulation is dispensed from the reservoir in a metered dose.

Preferably each dispensing outlet extends laterally from an upper part of each reservoir of the package which may be a cap or closure member, and preferably the reservoir is situated at a lower part.

Preferably the terminating portion of the static dispensing outlet is orientated at angle from 90 -135 degrees with respect to the longitudinal axis of the package.

The package may comprise a polymeric material selected from polypropylene (PP), polyethylene (PE), polycarbonate (PC), polyamides (PA) polyethylene terephthalate (PET), polyvinylchloride (PVC), polystyrene (PS), and combinations thereof, more preferably polypropylene, (PP), polyethylene (PE), polyethylene terephthalate (PET) or combinations thereof, most preferably the containers comprise polyethylene terephthalate.

If separate containers are used the material that the containers comprise may be selected independently of each other.

Preferably the containers are re-sealable. In the context of the present invention the term re-sealable means that the containers may be re-sealed after use and so are suitable for more than one application of product.

Preferably the pump dispenser is a non-aerosol device. Preferably the pump dispenser comprises a manually operated unidirectional reciprocated pump dispenser.

Preferably the dispenser dispenses a calibrated volume, preferably in the range from 1 to 10ml. Preferably the ratio of the volume dispensed from the first reservoir to the volume dispensed from the second reservoir is from 1:1 to 1:3. Most preferably the dispensing ratio is the same as the volume ratio of the two reservoirs.

<u>Polyphenol Formulation</u>

The first composition comprising a polyphenol. The polyphenol of the invention comprises at least one benzene ring substituted with a first OH group and a second OH or $OCH_3$ group, preferably OH. Preferably the first OH is ortho to the second OH group.

The OH containing benzene ring may be fused to further aromatic or non-aromatic rings.

Polyphenols may be found in the classes of phenolic acid, Anthocyanin, Anthocyanidin, flavanols, flavanones and gallates. For example flavan-3-ols, flavan-4-ols, flavan-3,4-ols. Gallic Acid, flavanols and flavanones are preferred.

A preferred polyphenol contains a benzene ring substituted in the: 1 position by an X group, wherein X is a carbon containing organic group, preferably selected from an ester group, COOH, chromenes and 3,4 dihydrochromenes. 3 position by a H, OH or $OCH_3$ group, preferably an OH group. 4 position by an OH group. 5 position by an OH or $OCH_3$ group, preferably an OH group.

Most preferably the 2 and 6 positions are substituted by H.

Preferably the polyphenol is a ortho-dihydroxybenzene derivative.

Preferably the polyphenol has a molecular weight of less than 1,000, more preferably less than 500.

Polyphenols may be suitably found in the wood and bark of trees and in the fruit, seeds and nuts of plants. For example the leaves of *Camilla senesis*; the wood of *Haematoxylum campechianum*; the bark of *Quercus velutina*; the fruit *of Vitis vinifer;* the fruit <u>*of Olea europaea*</u>*;* the roots *of Curcuma demoestica*; the trunk and bark of *Quercus velutina* and *Quercus lusitanica;* the trunk and bark of trees of the <u>*genus*</u> *Pseudotsuga;* the trunk and bark of *Acacia catechu.*

The polyphenol of the invention is preferably selected from gallic acid, methyl gallate, ethyl gallate, propyl gallate or mixtures thereof, most preferably propyl gallate

Methyl gallate, ethyl gallate, propyl gallate are obtainable by esterification of gallic acid. Preferably the gallic acid is extracted from a natural source, preferably from hydrolysable tannins, preferably extracted from tara plants or gallnuts. Preferably, the polyphenols do not contain any active oxidase enzymes and have been heat treated to destroy any enzyme activity.

The polyphenol is preferably selected from methyl gallate, ethyl gallate and propyl gallate.

The polyphenol containing composition is preferably an aqueous polyphenol solution. The polyphenol composition preferably comprises from 0.05 wt% to 10.0 wt% of the total shampoo composition, more preferably from 0.1 wt% to 5.0 wt %, most preferably 0.2 wt% to 3.0 wt% of polyphenol.

Wherein the polyphenol solution preferably has a pH at 20 °C of from 2 to 8 measured using a calibrated pH meter more preferably from preferably 3 to 7, most preferably from 3.5 to 6.5.

The aqueous polyphenol solution contains water, preferably as the dominant ingredient. Auxiliary ingredients may be present for example to increase the viscosity, perfume and help solubilise the polyphenol. Solubilising ingredients include organic solvents and surfactants.

Preferably, the water used to formulate all compositions has a French hardness of from 0 to 36 degrees, more preferably 0 to 24 degrees, most preferably from 0 to 2 degrees.

Preferably, the water used to formulate all compositions contains less than 2ppm of chlorine based bleaching agents such as chlorine dioxide or hypochlorite. Most preferably less than 200ppb

Compositions of the invention are generally aqueous, i.e. they have water or an aqueous solution or a lyotropic liquid crystalline phase as their major component. Suitably, the composition will comprise from 50% to 98%, preferably from 60% to 90% water by weight based on the total weight of the composition.

Preferably the polyphenol containing composition is a shampoo composition.

Cleansing Surfactant

Shampoo compositions according to the invention will generally comprise one or more cleansing surfactants which are cosmetically acceptable and suitable for topical application to the hair. The cleansing surfactant is preferably an anionic cleansing surfactant.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially sodium, magnesium, ammonium and mono-, di- and triethanolamine salts. The alkyl and acyl groups generally contain from 8 to 18, preferably from 10 to 16 carbon atoms and may be unsaturated. The al kyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate(n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5% to 45%, preferably from 1.5% to 35%, more preferably from 5% to 20% by total weight anionic cleansing surfactant based on the total weight of the composition.

The composition can further include co-surfactants, to help impart aesthetic, physical or cleansing properties to the composition.

An example of a co-surfactant is a nonionic surfactant, which can be included in an amount ranging from 0.5% to 8%, preferably from 1.0% to 5.0% by weight based on the total weight of the composition.

For example, representative nonionic surfactants that can be included in shampoo compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide.

Further nonionic surfactants which can be included in shampoo compositions of the invention are the alkyl polyglycosides (APGs). Typically, APG is one which comprises an alkyl group connected (optionally via a bridging group) to a block of one or more glycosyl groups. Preferred APGs are defined by the following formula:

$$RO - (G)_n$$

wherein R is a branched or straight chain alkyl group which may be saturated or unsaturated and G is a saccharide group.

R may represent a mean alkyl chain length of from about $C_5$ to about $C_{20}$. Preferably R represents a mean alkyl chain length of from about $C_8$ to about $C_{12}$. Most preferably the value of R lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the $C_{10}$-$C_{18}$ N-alkyl ($C_1$-$C_6$) polyhydroxy fatty acid amides, such as the $C_{12}$-$C_{18}$ N-methyl glucamides, as described for example in WO 92 06154 and US 5 194 639, and the N-alkoxy polyhydroxy fatty acid amides, such as $C_{10}$-$C_{18}$ N-(3-methoxypropyl) glucamide.

A preferred example of a co-surfactant is an amphoteric or zwitterionic surfactant, which can be included in an amount ranging from 0.5% to about 8%, preferably from 1 % to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocoamidopropyl betaine.

Mixtures of any of the foregoing amphoteric or zwitterionic surfactants may also be suitable. Preferred mixtures are those of cocoamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1% to 50%, preferably from 2% to 40%, more preferably from 10% to 25% by total weight surfactant based on the total weight of the composition.

Polyacrylic acid

The polymers of the invention may polymers comprising polyacrylic acids. Such polymers are preferably polyacrylic acids per se, cross-linked polymers of acrylic acid, copolymers of acrylic acid with a hydrophobic monomer, copolymers of carboxylic acid-containing monomers and acrylic esters, cross-linked copolymers of acrylic acid and acrylate esters.

The weight average molecular weight of the polymer is preferably greater than 10,000, more preferably greater than 100,000, most preferably greater than 1,000,000.

The polymer preferably contains a mixture of acrylic acid and a crosslinker, preferably allyl sucrose or allyl pentaerythritol. A crosslinker is a compound containing 2 or more polymerisable double bonds, preferably acrylate groups. Most preferably 3 to 6. Preferably the mol ratio of acrylic acid to crosslinker is 850:150 to 999:1, more preferably 98:2 to 99:1.

Alkyl methyacrylate are optionally present, preferably, methyl methacrylate, ethyl methacrylate, propyl methacrylate and butyl methacrylate.

The polymerisation is preferably accomplished via free radical polymerisation.

Preferably the polymer is water miscible and at 0.5 wt% in demineralised water has a zero shear viscosity of greater than10,000 Pa.s (at shear stress of 1 Pa).

Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Lubrizol.

Suitable cross-linked polymers of acrylic acid and acrylate esters are Pemulen TR1 or Pemulen TR2.

Preferably the level of polyacrylic acid polymer in the shampoo is from 0.05 to 2 wt% of the shampoo composition, more preferably from 0.1 wt% to 1 wt%, most 0.2 to 0.6 wt%.

Cationic Polymers

Compositions of the invention may comprise cationic polymers. Suitable cationic polymers may be homopolymers which are cationically substituted or may be formed from two or more types of monomers. The weight average ($M_w$) molecular weight of the polymers will generally be between 100,000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via

the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;

- mineral acid salts of amino-alkyl esters of homo-and co-polymers of unsaturated carboxylic acids having from 3 to 5 carbon atoms, (as described in U.S. Patent 4,009,256);

- cationic polyacrylamides (as described in WO95/22311).

Other cationic polymers that can be used include cationic polysaccharide polymers, such as cationic cellulose derivatives, cationic starch derivatives, and cationic guar gum derivatives. Cationic polysaccharides are particularly preferred as they mitigate the staining of fabrics.

Cationic polysaccharide polymers suitable for use in compositions of the invention include monomers of the formula:

$$A\text{-}O\text{-}[R\text{-}N^{+}(R^{1})(R^{2})(R^{3})X^{-}],$$

wherein: A is an anhydroglucose residual group, such as a starch or cellulose anhydroglucose residual. R is an alkylene, oxyalkylene, polyoxyalkylene, or hydroxyalkylene group, or combination thereof. $R^1$, $R^2$ and $R^3$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^1$, $R^2$ and $R^3$) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 % to 5%, preferably from 0.05 % to 1 %, more preferably from 0.08 % to 0.5% by total weight of cationic polymer based on the total weight of the composition.

<u>Suspending Agent</u>

Shampoo compositions of the invention may comprise suspending agents. Ethylene glycol distearate and polyethylene glycol 3 distearate are preferred long chain acyl derivatives, since these impart pearlescence to the composition. , heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. A suitable heteropolysaccharide gum is xanthan gum, for example that available as Kelzan mu.

Mixtures of any of the above suspending agents may be used.

Suspending agent will generally be present in a shampoo composition of the invention at levels of from 0.1 % to 10%, preferably from 0.5% to 6%, more preferably from 0.9% to 4% by total weight of suspending agent based on the total weight of the composition.

Further Ingredients

Optionally, a shampoo composition of the invention may contain further ingredients as described below to enhance performance and/or consumer acceptability.

Product Form

Compositions of the invention are typically "rinse-off' compositions to be applied to the hair and then rinsed away.

Metal containing composition

Metal Complex

Compositions of the invention comprise a metal ion preferably selected from an Fe(II), Fe(III) complex.

Metal ion containing compositions of the invention comprise 0.01 wt% of the total composition of a metal ion, preferably from 0.01 wt% to 10 wt%, more preferably from 0.1 wt% to 5 wt% of the total composition, more preferably from 0.2 to 3 wt%.

The level of metal ion in the formulation may be determined by quantitative elemental analysis.

For the avoidance of doubt, if the formulation contains 2 wt% of the complex

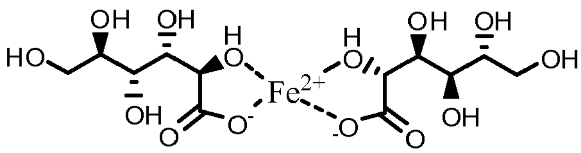

With molecular weight of 446.14, then it will contain 55.84/446.14*2 = 0.25 wt% of Fe(II) (2 decimal places).

The metal ion is present as a complex with a ligand. The ligand is preferably organic in nature.

In the context of this invention a ligand is a molecule that contains more than one group, preferably 2 to 4 groups, most preferably 2 groups that co-ordinate with the metal ion. Groups for co-ordinating with the metal ion are $COO^-$, OH, ketones, esters, primary amines, secondary amines and tertiary amines, more preferably $COO^-$, tertiary amines and OH, most preferably COO- and OH. Polyphenols are not permitted as multidentate ligands. The ligand-metal complex may be pre-formed before addition to the shampoo for example Iron (II) Gluconate, iron (II) glutamate. The ligand metal complex may be formed in the composition by the addition of an iron salt and the ligand, for example $FeCl_2$ and sodium lactate. Preferably, the mole ratio of iron ion to ligand is from 1:1 to 1:4, more preferably from 1:1.5 to 1:2.5.

Preferably, the metal ion is in the form of a complex that is soluble in aqueous solution at the pH of the formulation. Most preferably the metal ion in the form of a complex has a solubility in demineralised water at the pH of the formulation of greater than 0.1 g/L.

The ability of a ligand to complex with a metal ion, $M^{n+}$, may be defined by its $pM^{n+}$ value, wherein

$$pM^{n+} = -log_{10}[M^{n+}]_{free}$$

and the ligand concentration is $10^{-5}$ mol/L and the total metal ion concentration is $10^{-6}$ mol/L and $[M^{n+}]_{free}$ is the molar concentration of uncomplexed metal ions. Preferably the ligand used has a lower $pM^{n+}$, than gallic acid. Preferably the $pM^{n+}$ are greater than 8 more preferably greater than 10. $pM^{n+}$ values are most preferably measured at pH=4 in demineralised water, with the chloride salt of the metal ion, most preferably ferric chloride.

Preferably polyamino carboxylic acids are not the main ligand and polyamino carboxylic acids are preferably present at weight % levels of less than 1/30th of the main ligand, preferably they are absent from the conditioner. A polyamino carboxylic acid is a compound containing two or more amines connected through carbon atoms to two or more carboxylic acid groups. 2,2',2",2"'-(Ethane-1,2-diyldinitrilo)tetraacetic acid is a polyamino carboxylic acid. Diethylene triamine pentaacetic acid is a polyamino carboxylic acid. Ethylenediamine-*N,N'*-disuccinic acid is a polyamino carboxylic acid.

Most preferably the ligands are selected from gluconate, tartrate, ascorbate, citrate and lactate. Particularly preferred are gluconate, lactate or ascorbate.

Particularly preferred are iron (II) gluconate, iron (III) gluconate, iron (II) lactate, and iron (III) lactate, most preferably iron (II) gluconate and iron (III) gluconate.

Preferably the metal containing composition is a conditioning composition. Such conditioner compositions will typically comprise one or more conditioning surfactants which are cosmetically acceptable and suitable for topical application to the hair.

Suitable conditioning surfactants include those selected from cationic surfactants, used singly or in admixture. Preferably, the cationic surfactants have the formula $N^+R^1R^2R^3R^4$ wherein $R^1$, $R^2$, $R^3$ and $R^4$ are independently ($C_1$ to $C_{30}$) alkyl or benzyl. Preferably, one, two or three of $R^1$, $R^2$, $R^3$ and $R^4$ are independently ($C_4$ to $C_{30}$) alkyl and the other $R^1$, $R^2$, $R^3$ and $R^4$ group or groups are ($C_1$-$C_6$) alkyl or benzyl. More preferably, one or two of $R^1$, $R^2$, $R^3$ and $R^4$ are independently ($C_6$ to $C_{30}$) alkyl and the other $R^1$, $R^2$, $R^3$ and $R^4$ groups are ($C_1$-$C_6$) alkyl or benzyl groups. Optionally, the alkyl groups may comprise one or more ester (-OCO- or - COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include the cationic surfactant is selected from cetyltrimethylammonium chloride, behenyltrimethylammonium chloride, cetylpyridinium chloride, dodecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride, cocotrimethylammonium chloride, PEG-2-oleammonium chloride and the corresponding hydroxides thereof.

Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:

(i) an amidoamine corresponding to the general formula (I):

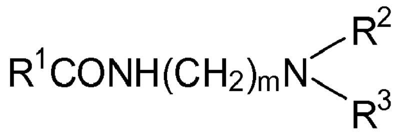

in which $R^1$ is a hydrocarbyl chain having 10 or more carbon atoms,
$R^2$ and $R^3$ are independently selected from hydrocarbyl chains of from 1 to 10 carbon atoms, and
m is an integer from 1 to about 10; and

(ii) an acid.

As used herein, the term hydrocarbyl chain means an alkyl or alkenyl chain.

Preferred amidoamine compounds are those corresponding to formula (I) in which $R^1$ is a hydrocarbyl residue having from about 11 to about 24 carbon atoms, $R^2$ and $R^3$ are each independently hydrocarbyl residues, preferably alkyl groups, having from 1 to about 4 carbon atoms, and m is an integer from 1 to about 4.

Preferably, $R^2$ and $R^3$ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

It especially preferred conditioners of the invention the cationic surfactant is selected from cetyltrimethylammonium chloride, behenyltrimethylammonium chloride or mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

A protonating acid may be present. Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof.

Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

In conditioners of the invention, the level of cationic surfactant will generally range from 0.01% to 10%, more preferably 0.1% to 7.5%, most preferably 0.2% to 5% by weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 % to 10%, preferably from 0.1 % to 8%, more preferably from 0.2% to 7%, most preferably from 0.3% to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

<u>Product form</u>

To avoid oxidation of the metal by air it is preferred that the composition is stored in an air tight container such as a bottle closed with an air tight cap.

<u>Further Conditioning Agents</u>

Compositions of the invention; shampoos or conditioners; may comprise further conditioning agents to optimise wet and dry conditioning benefits.

The compositions of the invention can contain, emulsified droplets of a silicone conditioning agent, for enhancing conditioning performance. Silicone conditioning agents may be present in the shampoo or conditioner.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed $10^9$ cst for ease of formulation.

Emulsified silicones for use in the shampoo compositions of the invention will typically have an average silicone droplet size in the composition of less than 30 $\mu$m, preferably less than 20 $\mu$m, more preferably less than 10 $\mu$m, ideally from 0.01 $\mu$m to 1 $\mu$m. Silicone emulsions having an average silicone droplet size of $\leq$ 0.15 $\mu$m are generally termed

microemulsions.

Emulsified silicones for use in the conditioner compositions of the invention will typically have an size in the composition of less than 30 μm, preferably less than 20 μm, more preferably less than 15 μm. Preferably, the average silicone droplet is greater than 0.5 μm, more preferably greater than 1 μm, ideally from 2 μm to 8 μm.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone", Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

With some shampoos it is preferred to use a combination of amino and non amino functional silicones

The total amount of silicone is preferably from 0.01 wt% to 10 wt% of the total composition more preferably from 0.1 wt% to 5 wt%, most preferably 0.5 wt% to 3 wt% is a suitable level, especially for a shampoo composition.

<u>Other Optional Ingredients</u>

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

<u>Method of use</u>

The method of colouring hair comprising the steps of applying to hair sequentially applying to the

(i) a first composition as described above followed by
(ii) a second composition as described above.

Preferably, the compositions of the invention are applied to wet hair, necessitating the step of wetting the hair before application of the compositions of the invention.

It is highly preferred if the hair is rinsed after application of both compositions of the invention.

Further conditioning and/or styling products may be applied as part of the colouring process.

The level of each composition applied to the head of hair is preferably from 5g to 100g. preferabley the dispensing outlet will be capable of dispensig this amount in a measured way.

Preferably, each composition remains on the hair for 5 to 600 seconds, more preferably 10 to 300 seconds.

Preferably, the water used to wet and rinse the hair has a French hardness of from 0 to 36 degrees, more preferably 0 to 24 degrees, most preferably from 0 to 2 degrees.

Preferably, the water used to wet and rinse the hair contains less than 2ppm of chlorine based bleaching agents such as chlorine dioxide or hypochlorite. Most preferably less than 200ppb.

Figure 1 shows an enlarged schematic view of a package according to the invention.

Referring to the drawings, a packaged hair colorant kit referred to generally at 1 comprising a pump package 2 in which is contained two compositions 3 and 4 for colouring hair according to the example herein below is shown.

The pack comprises two reservoirs 5 and 6 in which the compositions 3 and 4 are contained and two pump dispensers 7 and 8 by which the compositions 3 and 4 are respectively dispensed from the reservoirs 5 and 6 dispensing outlet nozzles10 and 11. The nozzles 10 and 11 extends laterally from the main body of the cap 12 Each nozzle is positioned at an angle of 180 degrees on a horizontal plane from the opposing nozzle.

Non-limiting Example of the compositions invention are as follows:

All levels are in wt% of the total composition

Composition 1

| | Shampoo 1 |
|---|---|
| Propyl Gallate | 0.3 |
| Surfactant | 13.6 |
| structurant | 9 |
| preservatives | 0.14 |
| water | To 100 |

The surfactant used was Sodium lauryl ether sulphate (1 EO) and cocoamidopropyl betaine in the ratio 15:2.

The structurant used was carbopol 980 (ex Lubrizol).

Composition 2

An iron containing conditioner formulation was also made of the following composition.

| Ingredient | Weight% |
|---|---|
| Behentrimonium Chloride | 1.13 |
| Stearamidopropyldimethylamine | 0.38 |
| Cetearyl Alcohol | 4 |
| Lactic Acid | 0.10 |
| Preservative | 0.09 |
| Iron (II) gluconate | 4.00 |
| Water | To 100 |

## Claims

1. A kit for colouring hair comprising at least two compositions and a package containing the compositions in which:

   a) the package comprises:

      (i) a separate reservoir for each composition
      (ii) a dispensing outlet for dispensing a first composition from a first reservoir and a second dispensing outlet for dispensing a second composition from a second reservoir; and in which
      (iii) the dispensing outlet of the first reservoir is positioned at an angle from 90 to 270 degrees on a horizontal plane from the dispensing outlet of the second reservoir;

   b) the compositions comprise:

      (i) a first composition comprising a polyphenol based formulation;
      (ii) a second composition comprising a metal ion based formulation in which the first composition is contained within the first reservoir and the second composition is contained within the second reservoir.

2. A kit according to claim 1 in which the dispensing outlet of the first reservoir is positioned at an angle from 160 to 200 degrees on a horizontal plane from the dispensing outlet of the second reservoir.

3. A kit according to any preceding claim in which the first dispensing outlet is static relative to the second dispensing outlet.

**4.** A kit according to any preceding claim in which each dispensing outlet comprises a hand-operated pump actuator by which each composition is dispensed from each reservoir in a metered dose.

**5.** A kit according to any preceding claim wherein each dispensing outlet extends laterally from an upper part of the package.

**6.** A kit according to any preceding claim where the terminating portion of the dispensing outlet is orientated at angle from 90 -135 degrees with respect to the longitudinal axis of the package.

**7.** A kit according to any preceding claim in which the package comprise (PP), polyethylene (PE), polyethylene terephthalate (PET) or combinations thereof.

**8.** A kit according to any preceding claim in which

(i) the first composition comprises

a) a cleansing surfactant and;
b) a polyphenol;

ii) the second composition comprises

a) a cationic surfactant; and
b) at least 0.01 wt% of a metal ion .

**9.** A kit according to any preceding claim in which the metal ion is selected from Fe(II) and/or Fe(III) complex.

**10.** A kit according to any preceding claim in which the iron is present as iron (II) gluconate or/and iron (III) gluconate.

**11.** A kit according to any preceding claim in which the polyphenol is selected from gallic acid, methyl gallate, ethyl gallate, propyl gallate or mixtures thereof.

**12.** A kit according in claim 10 in which the polyphenol is propyl gallate.

**13.** A kit according to any preceding claim in which the cationic surfactant is selected from cetyltrimethylammonium chloride, behenyltrimethylammonium chloride or mixtures thereof.

**14.** A process for colouring hair, the process using the kit of any of the preceding claims, the process comprising the steps of:

(i) dispensing a first composition from the first reservoir using the first dispensing outlet;
ii) applying the composition to the hair;
iii) rinsing the hair;
iv) dispensing a second composition from the second reservoir using the second dispensing outlet;
v) applying the composition to the hair;
vi) rinsing the hair.

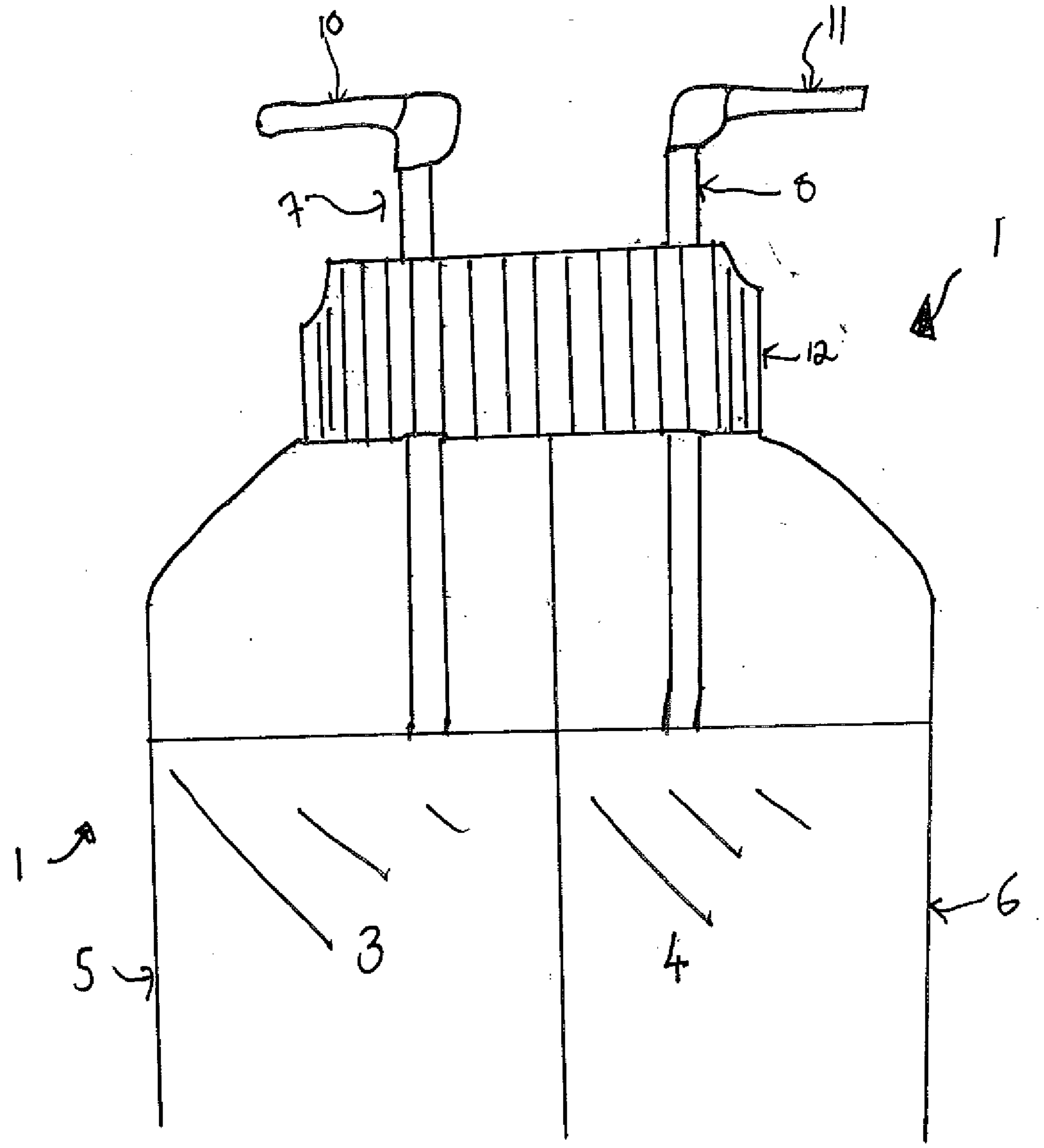
10
11
7
8
1
12
1
3
4
5
6

Europäisches Patentamt
European Patent Office
Office européen des brevets

# EUROPEAN SEARCH REPORT

Application Number
EP 12 18 3441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2010 248103 A (SHISEI CHEMICAL KK) 4 November 2010 (2010-11-04) * paragraphs [0002], [0003], [0021], [0027], [0034], [0035]; claims; examples * | 1-14 | INV. A45D19/00 A61K8/19 A61K8/34 A61K8/37 A61Q5/10 |
| X | CN 101 812 246 A (ZHEJIANG YANGSHENGTANG NATURAL MEDICINES INST CO LTD) 25 August 2010 (2010-08-25) * paragraphs [0002], [0041], [0052], [0082], [0111]; claims; examples * | 1-14 | |
| X | WO 96/02162 A1 (ANTHONY BERNARD INC [US]) 1 February 1996 (1996-02-01) * page 1, lines 7,20-23 * * page 5, lines 1-6; figures * | 1-13 | |
| X | KR 101 177 827 B1 (KWANGDUK SINYOUK [KR]) 28 August 2012 (2012-08-28) * the whole document * | 1-13 | |
| | | | TECHNICAL FIELDS SEARCHED (IPC) |
| | | | A45D A61K A61Q |

The present search report has been drawn up for all claims

| Place of search | Date of completion of the search | Examiner |
|---|---|---|
| Munich | 15 March 2013 | Mitchell, Gemma |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

## ANNEX TO THE EUROPEAN SEARCH REPORT ON EUROPEAN PATENT APPLICATION NO.

EP 12 18 3441

This annex lists the patent family members relating to the patent documents cited in the above-mentioned European search report.
The members are as contained in the European Patent Office EDP file on
The European Patent Office is in no way liable for these particulars which are merely given for the purpose of information.

15-03-2013

| Patent document cited in search report | Publication date | Patent family member(s) | Publication date |
|---|---|---|---|
| JP 2010248103 A | 04-11-2010 | NONE | |
| CN 101812246 A | 25-08-2010 | NONE | |
| WO 9602162 A1 | 01-02-1996 | US 5554197 A | 10-09-1996 |
| | | WO 9602162 A1 | 01-02-1996 |
| KR 101177827 B1 | 28-08-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9206154 A
- US 5194639 A
- US 4009256 A
- WO 9522311 A
- US 3962418 A
- US 3958581 A
- WO 9631188 A
- EP 0530974 A